# EUROPEAN PATENT APPLICATION

(11) **EP 4 818 152 A1**
(43) Date of publication of application: **07.10.2026**
(21) Application number: 24898086.4
(22) Date of filing: 26.11.2024
(51) Int. Cl.: C07C 227/18, C07C 229/22, A61K 31/198, A61P 25/00, A61P 25/18, A61P 25/28, A61P 25/24, A23L 33/175

(54) **METHOD FOR PREPARING L-SERINE HYDROCHLORIDE AND USE THEREOF**

(30) Priority: 29.11.2023 KR 20230169195
(71) Applicant: Astrogen, Inc., Daegu 41072 (KR)
(72) Inventor: RYU, Hyung Chul, Daegu 41072 (KR); HWANG, Su-Kyeong, Daegu 41072 (KR); KIM, Hyung Jun, Daegu 41072 (KR); KANG, Hee-Geun, Daegu 41072 (KR)
(74) Representative: Dehns
(86) International application number: PCT/KR2024/018899
(87) International publication number: WO 2025/116492

(57) **Abstract**

The present invention relates to a method for preparing an L-serine hydrochloride and a use of the L-serine hydrochloride prepared by the preparation method. Specifically, the L-serine hydrochloride according to the present invention has increased water solubility and bioavailability and improved storage stability, is convenient for preparing a composition, and has a superior pharmacological profile. The L-serine hydrochloride can be used for preventing, alleviating, or treating neurological, psychiatric, and cognitive disorders.

## Description

### Technical Field

The present invention relates to a method for preparing L-serine hydrochloride and a use of L-serine hydrochloride prepared by the method.

### Background Art

In general, when preparing pharmaceutical compositions, physicochemical stability and solubility are crucial factors that determine processing costs and the pharmacokinetics of the product prepared. In particular, solution stability and rapid solubility are essential for the preparation of syrup-type or rapid-disintegrating tablets.

Active pharmaceutical ingredients (APIs) are prepared and administered as salts or co-crystals to improve purification, facilitate the preparation process through easy preparation of crystalline powders, enhance solubility, and increase bioavailability. The selection of materials used in the preparation of these salts or co-crystals must be carefully considered based on the amount of API used.

Korean Patent No. KR10-1948066B1 relates to a composition for preventing, improving, or treating neurodevelopmental disorders, which contains L-serine as an active ingredient. In this prior art document, the amount of L-serine administered for effective treatment was 200-400 mg/kg, and a relatively large amount of serine was administered twice a day.

Meanwhile, Korean Patent Application Publication No. KR10-2022-0131186 relates to a syrup, which is an optimal drug formulation that can be consumed by children with autism spectrum disorder, in which L-serine used in the preparation of the syrup of the above-mentioned prior art document is an amino acid in the form of a free base in a natural state. Additionally, according to the above-mentioned prior art document, it appears to have a high water solubility of 425 mg/mL; however, in reality, it has a low water solubility of about 100 mg/mL due to the use of a sweetener such as sorbitol, which is used together with the syrup to ensure the convenience of taking the drug for children. This means that, for example, the amount of L-serine to be administered for a 30 kg pediatric patient for treatment is about 12,000 mg (30 × 400 mg) per day, and if converted to the amount of syrup, about 120 mL of syrup needs to be consumed. This is a significantly large dose to administer to children with autism spectrum disorder, who are typically sensitive and difficult to control.

Meanwhile, when the preparation is performed by reducing the amount of sweetener or increasing the temperature to improve solubility, not only does it show low medication compliance due to change in taste, but storage stability is very poor due to precipitation of L-serine when the temperature is lowered, and there is a problem in that browning occurs when the drug is stored in a solution state for a long period of time.

In addition, although L-serine hydrochloride is sold by some reagent companies such as Aldrich, there is no known economical industrial preparation process for producing hydrochloride, and only a small amount of L-serine hydrochloride is sold, which is not suitable for medical use.

Accordingly, in order to solve the above-mentioned problems, the present inventors have developed a novel hydrochloride of L-serine and a method for preparing the same, which enables the preparation of a high-content syrup by increasing the solubility, does not discolor even when the drug is stored in an aqueous solution for a long period of time, and has an increased half-life and maximum absorption concentration when administered orally, thereby enabling effective treatment or improvement with only one dose per day.

### Disclosure of Invention

### Technical Problem

An object of the present invention is to provide L-serine hydrochloride having increased water solubility and bioavailability, improved storage stability, suitability for preparation of compositions, and an improved pharmacological profile, as well as an economical method for preparing the same.

Another object of the present invention is to provide a use of L-serine hydrochloride prepared by the above method in the prevention, improvement, or treatment of neurological, mental, and cognitive disorders.

### Solution to Problem

In order to solve the above-mentioned problems, the present invention provides a method for preparing L-serine hydrochloride, which includes the following:
(a) mixing L-serine of Formula 1 below with an organic solvent;
(b) adjusting the pH of the mixed solution of step (a) above with hydrochloric acid diluted in an organic solvent;
(c) forming a salt by stirring the solution whose pH has been adjusted in step (b) above; and
(d) filtering the salt prepared in step (c) above and then washing the excess hydrochloric acid with an organic solvent

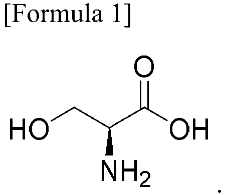

The present invention also provides L-serine hydrochloride of Formula 2 below prepared by the above preparation method.

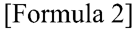

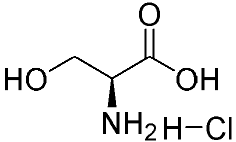

The present invention provides a pharmaceutical composition for the prevention or treatment of neurological, mental, and cognitive disorders, comprising L-serine hydrochloride as an active ingredient.

The present invention also provides a health functional food composition for the prevention or improvement of neurological, mental, and cognitive disorders, comprising L-serine hydrochloride as an active ingredient.

### Advantageous Effects of Invention

L-serine hydrochloride according to the present invention has increased water solubility and bioavailability, improved storage stability, suitability for preparation of compositions, and a superior pharmacological profile. Additionally, due to the increased half-life and maximum absorption concentration in a composition comprising L-serine hydrochloride according to the present invention, it is possible to effectively prevent, improve, or treat neurological, mental, and cognitive disorders with only one dose per day.

### Brief Description of Drawings

Fig. 1 shows differential scanning calorimetry (DSC) data of L-serine hydrochloride prepared in Example 3.
Fig. 2 shows powder X-ray diffraction (PXRD) data of L-serine hydrochloride prepared in Example 3.
Fig. 3 shows differential scanning calorimetry (DSC) data of L-serine.
Fig. 4 shows powder X-ray diffraction (PXRD) data of L-serine.
Fig. 5 shows the data obtained by titrating L-serine hydrochloride prepared in Example 3 with a silver nitrate solution (ERC: solid line, U: dotted line).
Fig. 6 shows the results of the pharmacokinetic evaluation of L-serine hydrochloride prepared in Example 3.

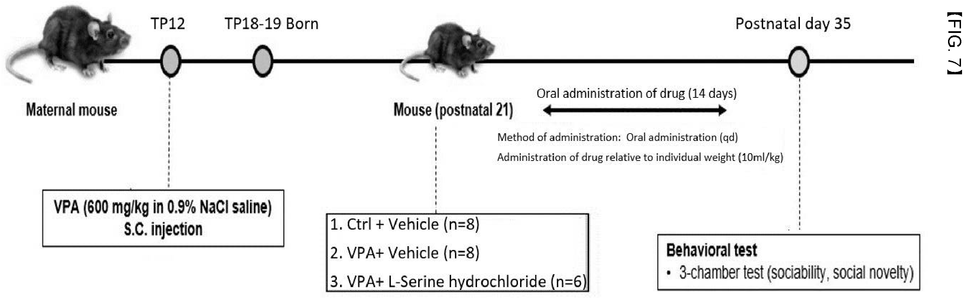

Fig. 7 shows the behavioral experiment procedure of valproic acid (VPA)-induced autism model mice using L-serine hydrochloride prepared in Example 3.
Figs. 8A and 8B show the results of a 3-chamber test that evaluated the social interaction ability of mice, in which Fig. 8A confirms social novelty, and Fig. 8B confirms sociability.

### Best Mode for Carrying out the Invention

Unless otherwise defined, all technical and scientific terms used herein have the same meaning as commonly understood by one of ordinary skill in the art to which this invention pertains. In general, the nomenclature used herein is well known and commonly used in the art.

The present invention relates, in one aspect, to a method for preparing L-serine hydrochloride.

An exemplary embodiment of the present invention provides a method for preparing L-serine hydrochloride, which includes:
(a) mixing L-serine of Formula 1 below with an organic solvent;
(b) adjusting the pH of the mixed solution of step (a) above with hydrochloric acid diluted in an organic solvent;
(c) forming a salt by stirring the solution whose pH has been adjusted in step (b) above; and
(d) filtering the salt prepared in step (c) above and then washing the excess hydrochloric acid with an organic solvent

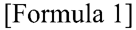

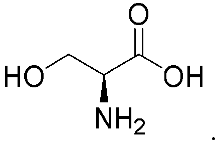

In the present invention, "L-serine", which is a type of amino acid constituting proteins, is a monoamide of glutamic acid, and means an L-amino acid having the structure of Formula 1 above.

In the present invention, step (a) is a step of mixing L-serine with an organic solvent.

The organic solvent used in step (a) may be selected from those known in the art, as long as it does not deviate from the purpose of the present invention. Specifically, the organic solvent may be one or more selected from the group consisting of acetone, methanol, ethanol, isopropanol, butanol, hexanol, ethyl acetate, toluene, methyl-*t*-butyl ether, and methylene chloride, preferably acetone, methanol, ethanol, or isopropanol, and most preferably isopropanol or ethanol, but is not limited thereto.

In addition, the organic solvent may be used in admixture with water in an amount of about 10-40% of the total amount of solvent to facilitate smooth stirring; however, when used in an amount exceeding this range, the reaction yield decreases and the process becomes uneconomical. The amount of the organic solvent may be about 6 to 30 times the weight of L-serine used, preferably about 5 to 20 times, and most preferably 3 to 6 times, but is not limited thereto. When an excessive amount of solvent is used, process handling becomes difficult, and the amount of wastewater to be discarded after the process increases; therefore, it is important to use a minimum amount to ensure economic feasibility. In addition, when the temperature is increased, a portion of L-serine is converted to D-serine; therefore, it is necessary not to raise the temperature above room temperature.

In the present invention, step (b) is a step of adjusting the pH of the mixed solution of step (a) above with hydrochloric acid diluted in an organic solvent.

When the solution is adjusted to an acidic pH, the solubility in water increases by more than 5 times compared to neutral L-serine, which can significantly reduce the process volume. The pH adjusted in step (b) can be about 1.0 to 5.0, preferably about 2.0 to 4.5, and most preferably about 2.5 to 4.0, but is not limited thereto. Meanwhile, when the pH is too low, the amount of acid required increases, thereby reducing economic feasibility; additionally, there is a risk of corrosion of process equipment, such as reactors or filters made of metals and labile to acids, and a possibility of side reactions, such as cross-esterification of additional solvents, which further reduces economic feasibility.

In step (b), hydrochloric acid diluted in an organic solvent is used. When concentrated hydrochloric acid is used undiluted, the temperature may rise excessively, causing some L-serine to be converted to D-serine. Therefore, the hydrochloric acid should be diluted about 1- to 10-fold with an organic solvent, preferably about 2- to 8-fold, and most preferably about 3- to 7-fold, and should be slowly added dropwise while maintaining the temperature at or below room temperature.

The organic solvent used in the hydrochloric acid dilution may be selected from those known in the art, as long as it does not deviate from the purpose of the present invention. Specifically, the organic solvent may be one or more selected from the group consisting of acetone, methanol, ethanol, isopropanol, butanol, hexanol, ethyl acetate, toluene, methyl-*t*-butyl ether, and methylene chloride, preferably acetone, methanol, ethanol, or isopropanol, and most preferably isopropanol or ethanol, but is not limited thereto. In addition, it is most preferable to use the same organic solvent as the organic solvent used in step (a), but is not limited thereto.

In the present invention, step (c) is a step of forming a salt by stirring the solution whose pH has been adjusted in step (b) above.

In step (c), the stirring time may be set so that the hydrochloride can be sufficiently formed. Specifically, the stirring may be performed for about 2 to 48 hours, preferably about 3 to 36 hours, and most preferably about 3 to 24 hours, but is not limited thereto.

In order to prevent the temperature from rising during stirring and the subsequent conversion of L-serine to D-serine, it is necessary to stir at a temperature at or below room temperature.

In the present invention, step (d) is a step of filtering the salt (solid) prepared in step (c) above and then washing excess hydrochloric acid with an organic solvent.

In step (d) above, filtration may be performed using any filtration method applicable to industrial production, such as reduced-pressure filtration, centrifugation, or filter drying.

In addition, the organic solvent used in the washing may be selected from those known in the art, as long as it does not deviate from the purpose of the present invention. Specifically, it may be one or more selected from the group consisting of acetone, methanol, ethanol, isopropanol, butanol, hexanol, ethyl acetate, toluene, methyl-t-butyl ether, methylene chloride, cyclohexane, and pentane, and most preferably acetone, methanol, ethanol, or isopropanol, but is not limited thereto. The amount of the organic solvent may be about 1 to 8 times that of L-serine, preferably about 2 to 7 times, and most preferably about 3 to 6 times. In particular, the organic solvent may have a moisture content of 1% or less in order to prevent a decrease in yield.

In the present invention, after step (d) above, step (e) of drying to remove the solvent may be further included.

In step (e) above, the drying temperature is performed using a vacuum or hot air drying method at about 20-80°C, preferably 30-70°C, and most preferably 40-60°C, but is not limited thereto. However, care must be taken as discoloration may occur when the drying temperature is too high.

In another aspect, the present invention relates to L-serine hydrochloride of Formula 2 below prepared by the preparation method above.

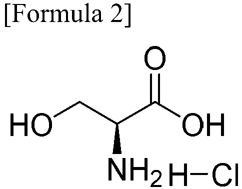

According to an embodiment of the present invention, crystalline L-serine hydrochloride may be formed.

The crystal form above may exhibit characteristic peaks at diffraction angles (2θ±0.2) of 16.4°, 16.9°, 18.8°, 19.9°, 20.7°, 22.5°, 25.3°, 26.6°, 27.2°, 27.8°, 29.0°, 30.8°, 32.4°, 33.1°, 37.5°, and 38.1° when measured in a powder X-ray diffraction (PXRD) analysis over a scanning range (2θ): 3° to 50°, and a scanning speed: 6 deg/min.

Additionally, the crystal form may exhibit a characteristic endothermic peak at about 150°C in the differential scanning calorimetry (DSC) thermogram.

In another aspect, the present invention relates to a pharmaceutical composition for the prevention or treatment of neurological, mental, and cognitive disorders, which comprises L-serine hydrochloride prepared by the preparation method above as an active ingredient.

As used herein, the term "treatment" refers to any act of improving or beneficially changing the symptoms of a disease by administering the composition according to the present invention, the term "prevention" refers to any act of inhibiting or delaying a disease by administering the composition according to the present invention, and the term "improvement" refers to any act of improving an adverse state of a disease by administering or ingesting the composition of the present invention to a subject.

The disease or condition targeted for "treatment", "prevention", or "improvement" of the present invention may be a neurological, mental, or cognitive disorder. The neurological, mental, or cognitive disorder is a general term for diseases that have abnormal brain function as their primary lesion, and may include autism spectrum, bipolar disorder, Tourette's syndrome, tics, Prader-Willi syndrome, attention deficit hyperactivity disorder (ADHD), fragile-X syndrome, Rett's syndrome, dementia, Alzheimer's disease, Parkinson's disease, schizophrenia, depression, and memory impairment, but is not limited thereto. For example, the L-serine hydrochloride may exhibit an effect of improving, delaying, preventing, or treating autism spectrum disorder, and more specifically, may exhibit an effect of improving, delaying, preventing, or treating childhood autism spectrum disorder.

For the treatment of the diseases or conditions described above, the compositions or preparations described herein may be administered orally. The term "oral" encompasses swallowing, and is used synonymously with administration by mouth. Oral administration allows the compounds of the present invention to enter the gastrointestinal tract, or, for example, to be absorbed directly into the bloodstream through the mouth, as in buccal or sublingual administration.

Suitable compositions for oral administration may be in the form of solids, liquids, gels, or powders, and may have dosage forms such as tablets, lozenges, capsules, granules, powders, and liquids.

Compositions for oral administration may optionally be enteric coated, and may exhibit delayed or sustained release through the enteric coating. That is, the compositions for oral administration according to the present invention may be formulations having an immediate or modified release pattern.

Liquid formulations may include solutions, syrups, and suspensions, and the liquid compositions may be contained within soft or hard capsules. The formulations may include pharmaceutically acceptable carriers, such as water, ethanol, polyethylene glycol, cellulose, or oil. The formulations may also include one or more emulsifiers and/or suspending agents.

More specifically, in the present invention, the syrup formulation refers to a concentrated liquid containing sugar or a sugar substitute, which is a medicine having an unpleasant taste, for example, a bitter taste, and is made into a liquid form for easy consumption. In particular, it is a formulation suitable for consumption by infants and children. The syrup may contain, in addition to purified water and an extract, an antibacterial preservative, a flavoring agent, a coloring agent, a sweetener, *etc*. used to provide sweetness and viscosity, but is not limited thereto. Examples of sweeteners that may be contained in the syrup include sucrose, mannitol, sorbitol, xylitol, aspartame, stevioside, fructose, lactose, sucralose, saccharin, or menthol, but are not limited thereto.

In tablet formulations, the active ingredient drug may be present in an amount of from about 0.05 wt% to about 95 wt% based on the total weight of the tablet, more typically from about 2 wt% to about 50 wt% of the formulation. The tablets may also contain a disintegrant, which accounts for about 0.5 wt% to about 35 wt%, more typically about 2 wt% to about 25 wt% of the formulation. Examples of disintegrants include lactose, starch, sodium starch glycolate, crospovidone, croscarmellose sodium, maltodextrin, or mixtures thereof, but are not limited thereto.

Suitable lubricants to be included for preparing tablets may be present in an amount of about 0.1 wt% to about 5 wt%, and examples of lubricants may include talc, silicon dioxide, stearic acid, calcium, zinc or magnesium stearate, sodium stearyl fumarate, *etc*., but are not limited thereto.

As binders for the preparation into tablets, gelatin, polyethylene glycol, sugar, gum, starch, polyvinylpyrrolidone, hydroxypropyl cellulose, hydroxypropyl methylcellulose, *etc*. may be used, and as suitable diluents for the preparation into tablets, mannitol, xylitol, lactose, dextrose, sucrose, sorbitol, starch, microcrystalline cellulose, *etc*. may be used, but are not limited thereto.

Solubilizing agents that may optionally be included in the tablet may be used in an amount of about 0.1 wt% to about 3 wt% based on the total weight of the tablet, and examples thereof may include polysorbate, sodium lauryl sulfate, sodium dodecyl sulfate, propylene carbonate, diethylene glycol monoethyl ether, dimethyl isosorbide, polyoxyethylene glycolated natural or hydrogenated castor oil, HCOR^{™} (Nikkol), oleyl ester, Gelucire^{™}, caprylic/caprylic acid mono/diglycerides, sorbitan fatty acid ester, Solutol HS^{™}, *etc*., but are not limited thereto.

In still another aspect, the present invention relates to a health functional food composition for the prevention or improvement of neurological, mental, and cognitive disorders, which comprises L-serine hydrochloride as an active ingredient.

In the present invention, "health functional food" refers to a food group that has added value by using physical, biochemical, bioengineering techniques to make the function of the food work and express it for a specific purpose, or a food that has been designed and processed so that the body's internal regulatory function regarding the regulation of biological defense rhythm, disease prevention, and recovery of the food composition is sufficiently expressed in the body. For the purpose of the present invention, the health functional food may refer to a health functional food for the improvement of neurological, mental, and cognitive disorders, for example, a health functional food for the prevention and improvement of memory loss.

The health functional food composition may be prepared using methods commonly used in the art, and may be prepared by adding raw materials and ingredients commonly added in the art during the preparation. Furthermore, the formulation of the health functional food composition may be prepared without limitation, as long as it is a formulation recognized as a food composition in the art.

Foods according to the present invention include, for example, various foods, beverages, gums, tea, vitamin complexes, functional foods, *etc*. Foods include, but are not limited to, special nutritional foods (*e.g*., formulated milk, infant and toddler food, *etc*.), processed meat products, fish products, tofus, jellies, noodles (*e.g*., ramens, noodles, *etc*.), breads, health supplementary foods, seasoned foods (*e.g*., soy sauce, soybean paste, red pepper paste, mixed paste, *etc*.), sauces, confectionery (*e.g*., snacks), candies, chocolates, gums, ice creams, processed dairy products (*e.g*., fermented milk, cheese, *etc*.), other processed foods, kimchi, pickled foods (*e.g*., various kimchi, pickled vegetables, *etc*.), beverages (*e.g*., fruit drinks, vegetable drinks, soy milks, fermented drinks, *etc*.), natural seasonings (*e.g*., ramen soup, *etc*.), food additives, *etc*. The foods, beverages, or food additives may be prepared by a conventional preparation method.

When the health functional food composition of the present invention is used as a health functional food additive, the composition may be added as is or used together with other health functional food ingredients, and may be used appropriately according to a conventional method. The mixing amount of the active ingredient may be appropriately determined depending on the intended use. Generally, when preparing a food or beverage, the composition of the present invention may be added in an amount of preferably about 50 parts by weight or less, more preferably 25 parts by weight or less, relative to the raw material. However, in the case of long-term intake for the purpose of health and hygiene or health control, the amount may be below the above range. In addition, the composition may be used in an amount greater than the above range as long as there is no problem in terms of safety.

The health functional food composition of the present invention may comprise, in addition to the hydrochloride of L-serine as an active ingredient, conventional food composition additives such as natural carbohydrates or flavoring agents as additional ingredients. Examples of the natural carbohydrates may include conventional sugars (*e.g*., monosaccharides such as glucose, fructose, *etc*.); disaccharides (*e.g*., maltose, sucrose, *etc*.); and polysaccharides (*e.g*., dextrin, cyclodextrin, *etc*.), and sugar alcohols (*e.g*., xylitol, sorbitol, erythritol, *etc*.). Examples of the flavoring agents may include natural flavoring agents (*e.g*., thaumatin), stevia extracts (*e.g*., rebaudioside A, glycyrrhizin, *etc*.), and synthetic flavoring agents (*e.g*., saccharin, aspartame, *etc*.).

In addition, the health functional food composition may include, in addition to the hydrochloride of L-serine, which is an active ingredient, nutrients, vitamins, minerals (electrolytes), flavoring agents such as synthetic flavoring agents and natural flavoring agents, coloring agents and thickening agents (cheese, chocolate, *etc*.), pectic acid and salts thereof, alginic acid and salts thereof, organic acids, protective colloid thickeners, pH adjusters, stabilizers, preservatives, glycerin, alcohol, carbonating agents used in carbonated beverages, *etc*. In addition, the food composition of the present invention may include fruit pulp for preparing natural fruit juice, fruit juice drinks, and vegetable drinks.

Any description that overlaps with the pharmaceutical composition of the present invention described above is omitted.

In still another aspect, the present invention relates to a method for the prevention or treatment of neurological, mental, and cognitive disorders in a subject, which includes administering L-serine hydrochloride to the subject.

As used herein, the term "subject" is used synonymously with an individual, and the subject may be a mammal, for example, a human, rat, cow, horse, pig, dog, sheep, goat, or cat.

Any description that overlaps with the pharmaceutical composition of the present invention described above is omitted.

In still another aspect, the present invention relates to a use of L-serine hydrochloride for the prevention or treatment of neurological, mental, and cognitive disorders.

Any description that overlaps with the pharmaceutical composition of the present invention described above is omitted.

In still another aspect, the present invention relates to a use of L-serine hydrochloride in the manufacture of a medicament for the prevention or treatment of neurological, mental, and cognitive disorders.

Any description that overlaps with the pharmaceutical composition of the present invention described above is omitted.

All matters mentioned in the compositions, treatment methods, and uses of the present invention apply equally unless they are contradictory.

Hereinafter, examples and other embodiments will be described in detail to aid understanding of the present invention. However, the examples according to the present invention may be modified in various different forms, and the scope of the present invention should not be construed as being limited to the examples described below. The examples of the present invention are provided to more fully explain the present invention to those with an ordinary skill in the art.

### Embodiments for Carrying Out the Invention

### Examples

### Example 1. Preparation of L-serine hydrochloride

4.0 kg of L-serine and 20 L of isopropyl alcohol (IPA) were placed in a reactor at room temperature and stirred for 10 minutes. Then, 12.6 kg of 3 M HCl in IPA was slowly added dropwise while maintaining room temperature. The mixture was stirred at room temperature for 12 hours, filtered, and washed with 8 L of IPA. Subsequently, the resultant was dried under vacuum at 50°C to obtain a white solid with a yield of 93.5%.

The obtained solid was dissolved in D₂O and observed by ¹H-NMR, and it was confirmed that there were three hydrogens in the characteristic peaks at 3.8 to 4.2.

### Example 2. Preparation of L-serine hydrochloride

4.0 kg of L-serine and 25 L of IPA were placed in a reactor at room temperature and stirred for 10 minutes. 9.1 kg of 4 M HCl in IPA was slowly added dropwise while maintaining room temperature. The mixture was stirred at room temperature for 12 hours, filtered, and washed with 8 L of IPA. Subsequently, the resultant was dried under vacuum at 50°C to obtain a white solid with a yield of 92.5%.

The obtained solid was dissolved in D₂O and observed by H-NMR, and it was confirmed that there were three hydrogens in the characteristic peaks at 3.8 to 4.2.

### Example 3. Preparation of L-serine hydrochloride

4.0 kg of L-serine and 25 L of IPA were placed in a reactor at room temperature and stirred for 10 minutes. 9.1 kg of 4 M HCl in IPA was slowly added dropwise while maintaining room temperature. The mixture was stirred for 12 hours at 10°C or lower, filtered, and washed with 8 L of IPA. Subsequently, the resultant was dried under vacuum at 50°C to obtain a white solid with a yield of 93.7%.

The obtained solid was dissolved in D₂O and observed by H-NMR, and it was confirmed that there were three hydrogens in the characteristic peaks at 3.8 to 4.2.

### Example 4. Preparation of L-serine hydrochloride

4.0 kg of L-serine and 25 L of ethanol were placed in a reactor at room temperature and stirred for 10 minutes. 9.0 kg of 4 M HCl in ethanol was slowly added dropwise while maintaining room temperature. The mixture was stirred for 12 hours at 10°C or lower, filtered, and washed with 8 L of IPA. Subsequently, the resultant was dried under vacuum at 50°C to obtain a white solid with a yield of 91.5%.

The obtained solid was dissolved in D₂O and observed by H-NMR, and it was confirmed that there were three hydrogens in the characteristic peaks at 3.8 to 4.2.

### Example 5. Preparation of L-serine hydrochloride

4.0 kg of L-serine and 25 L of ethanol were placed in a reactor at room temperature and stirred for 10 minutes. 9.0 kg of 4 M HCl in ethanol was slowly added dropwise while maintaining room temperature. The mixture was stirred for 12 hours at 10°C or lower, filtered, and washed with 8 L of acetone. Subsequently, the resultant was dried under vacuum at 50°C to obtain a white solid with a yield of 90.9%.

The obtained solid was dissolved in D₂O and observed by H-NMR, and it was confirmed that there were three hydrogens in the characteristic peaks at 3.8 to 4.2.

### Example 6-1. Differential Scanning Calorimeter (DSC) Analysis

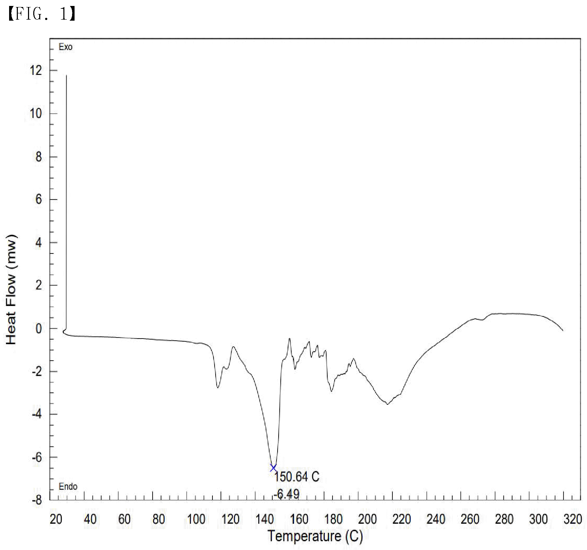

As a result of analyzing the L-serine hydrochloride prepared in Example 3 by differential scanning calorimetry (DSC), it was confirmed that crystalline L-serine hydrochloride was formed (Fig. 1). The formed crystalline L-serine hydrochloride is characterized by the peaks appearing in the DSC thermogram. The crystalline L-serine hydrochloride according to the present invention exhibited one endothermic peak at about 150°C (Fig. 1).

### Example 6-2. Powder X-Ray diffraction (PXRD) Analysis

Powder X-ray powder rotation patterns are widely used to distinguish between crystalline and hydrated forms of drugs due to their unique characteristics. Therefore, the powder X-ray pattern of L-serine hydrochloride was measured.

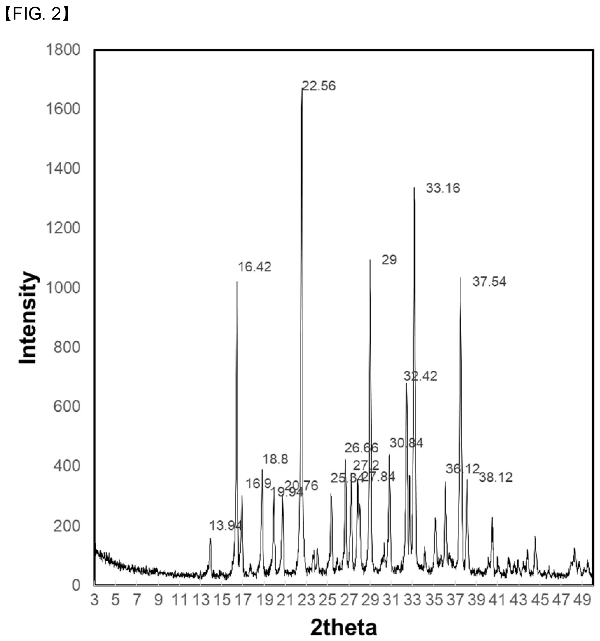

As a result of analyzing the L-serine hydrochloride prepared in Example 3 by powder X-ray diffraction (PXRD), it was confirmed that crystalline L-serine hydrochloride was formed (Fig. 2). The formed crystalline L-serine hydrochloride is characterized by the characteristic 2theta (2θ) diffraction angle peak appeared in PXRD.

Specifically, the crystalline L-serine hydrochloride according to the present invention exhibited characteristic peaks at diffraction angles (2θ±0.2) of 16.4°, 16.9°, 18.8°, 19.9°, 20.7°, 22.5°, 25.3°, 26.6°, 27.2°, 27.8°, 29.0°, 30.8°, 32.4°, 33.1°, 37.5°, and 38.1°, respectively, when measured in a PXRD analysis at a scanning range (2θ): 3° to 50° and a scanning speed: 6 deg/min (Fig. 2).

### Example 6-3. Comparison of L-serine hydrochloride and L-serine by Differential Scanning Calorimeter (DSC)

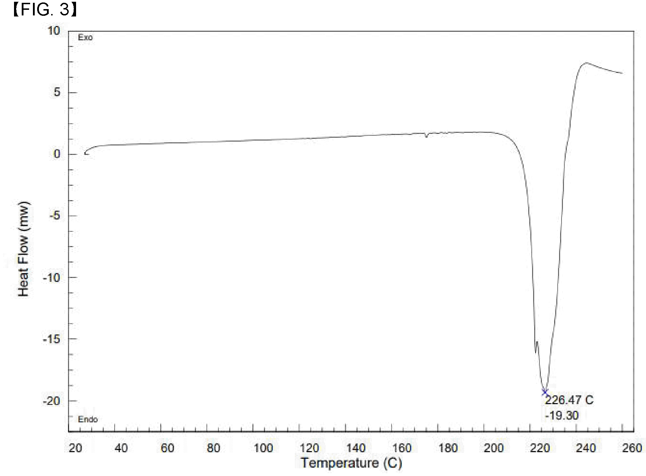

As a result of analyzing the L-serine hydrochloride prepared in Example 3 and L-serine by differential scanning calorimeter (DSC), the L-serine hydrochloride showed an endothermic peak at about 150°C as confirmed in Example 6-1, whereas L-serine showed an endothermic peak at about 226°C (Fig. 3).

### Example 6-4. Analytical Comparison of L-serine hydrochloride and L-serine by Powder X-Ray diffraction (PXRD)

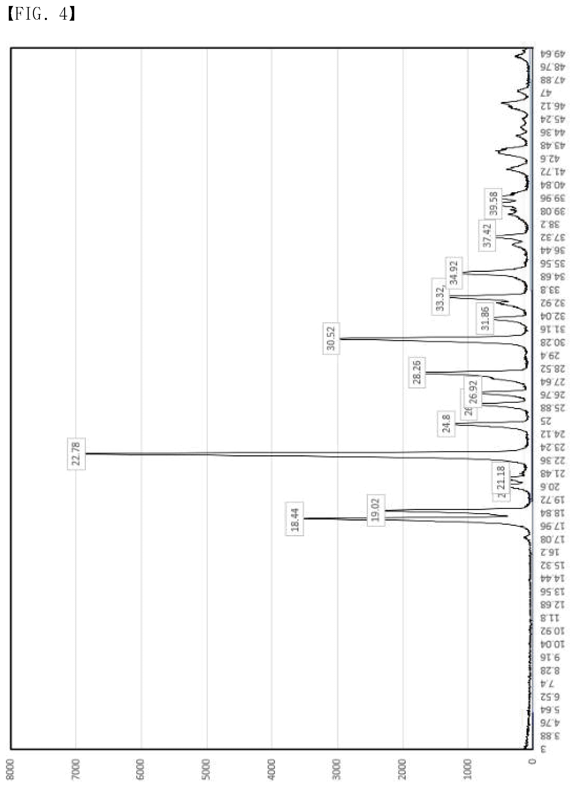

The powder X-ray diffraction (PXRD) angle values of L-serine had characteristic peaks at diffraction angles (2θ±0.2) of 18.4°, 19.0°, 18.8°, 19.9°, 20.7°, 22.8°, 24.8°, 26.9°, 28.2°, 30.5°, 31.8°, 33.3°, 34.9°, 37.4°, and 39.5°, respectively (Fig. 4).

As confirmed in Example 6-2, the numerical values of the powder X-ray diffraction (PXRD) angles of the L-serine hydrochloride were different from those of L-serine. Therefore, it was confirmed that the L-serine hydrochloride has a new crystalline form.

### Example 6-5. Chloride titration of L-serine hydrochloride

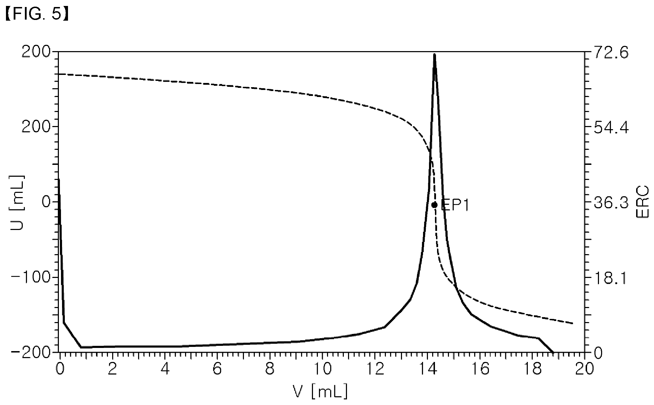

About 0.2 g of L-serine hydrochloride prepared in Example 3 was dissolved in water and titrated with a 0.1 mol/L silver nitrate solution, and the result showed that it contained about 22.5% of chloride (Fig. 5).

### Example 6-6. Comparison of Water Solubility between L-serine hydrochloride and L-serine

Water solubility was compared between the L-serine hydrochloride prepared in Example 3 and L-serine. While the water solubility of L-serine was 300 mg/mL, that of L-serine hydrochloride was 1560 mg/mL, confirming that the water solubility of the L-serine hydrochloride was increased at least 5-fold compared to that of L-serine. In addition, no discoloration or precipitation occurred even when the L-serine hydrochloride was stored in an aqueous solution at room temperature for one month.

Hereinafter, a mouse PK experiment was performed to confirm the improvement in the pharmacokinetic profile of the L-serine hydrochloride prepared in Example 3.

### Experimental Example 1

-6, -4, -2, and 0 hours before drug administration and 0.25, 0.5, 1, 3, 6, 9, and 12 hours after drug administration, the experimental mice were placed in a restrainer, a 2 mm incision was made at the tip of the tail, and about 40-50 µL of blood was collected into a heparin capillary tube while massaging the tail. After blood collection, the mice were euthanized in a CO₂ chamber and cervical dislocation was performed for disposal of the bodies. Blood samples were transferred to 2 mL tubes and centrifuged at 2000× g for 10 minutes to separate the serum, which was then transferred to new tubes at about 20-25 µL per sample. For LC-MS/MS analysis, 10 µL of serum was transferred to new tubes, and the remaining serum was stored in a deep freezer at -80°C. The L-serine hydrochloride prepared in Example 3 was diluted in water to a concentration of 10 mg/mL and the internal standard D, L-Serine-2,3,3-d3, was diluted in water to a concentration of 100 µg/mL. The L-serine hydrochloride solution was diluted in a solvent with a ratio of acetonitrile/water/trifluoroacetic acid (v/v/v, 96/5/0.5) to a concentration of 0.01 µg/mL to 41.38 µg/mL to prepare a standard reagent required for preparing a calibration curve. The internal standard D, L-serine-2,3,3-d3 stock solution was prepared at a concentration of 60 µg/mL for preparing a calibration curve and at 2 µg/mL for spiking into samples. To prepare the reagents required for creating the calibration curve, 10 µL of the diluted stock solution and 10 µL of the internal standard solution (D, L-serine-2,3,3-d3, 60 µg/mL) were spiked into 980 µL of a solvent with a ratio of acetonitrile/water/trifluoroacetic acid (v/v/v, 96/5/0.5). 10 µL of the rat plasma to be analyzed and 10 µL of the internal standard solution (D, L-serine-2,3,3-d3, 3.6 µg/mL) were spiked, and 980 µL of the initial condition solvent, acetonitrile/water/trifluoroacetic acid (v/v/v, 96/5/0.5), was added thereto, and the mixture was vortexed for 1 minute. The mixed solution was centrifuged at 16,000 g for 10 minutes, and the supernatant was separated and injected into LC-MS/MS at a volume of 5 µL. The ultra-high-performance liquid chromatography system consisted of a pump (LC-40D X3), an autosampler (SIL-40CX3), a column oven (CTO-40C), and a system controller (SCL-40) from Shimadzu (Duisburg, Germany), and the mass spectrometer used was a QTRQP LC-MS/MS 6500+ from SCIEX (Framingham, MA, USA). The column used was a CROWNPAK CR-I (+) [3.0 x 150 mm, 5 micron] from DAICEL (Osaka, Tokyo), and the column oven was set to 30°C. Acetonitrile/water/trifluoroacetic acid (v/v/v, 96/5/0.5) was used as the mobile phase, and the elution was performed under isocratic conditions. Mass spectrometry detection was performed under multiple reaction monitoring (MRM) conditions in ESI+ mode. MS/MS parameters were ion spray voltage, 5500 V; temperature 500°C; curtain gas, 30 psi; ion source gas (GS1), 50 psi, ion source gas (GS2), 50 psi. The declustering potential (DP), entrance potential (EP), collision energy (CE), and collision cell exit potential (CXP) values under MRM conditions were optimized in manual tuning mode using a syringe infusion pump, and mass spectrometric analysis was performed. Data acquisition and processing were performed using AB SCIEX Analyst software.

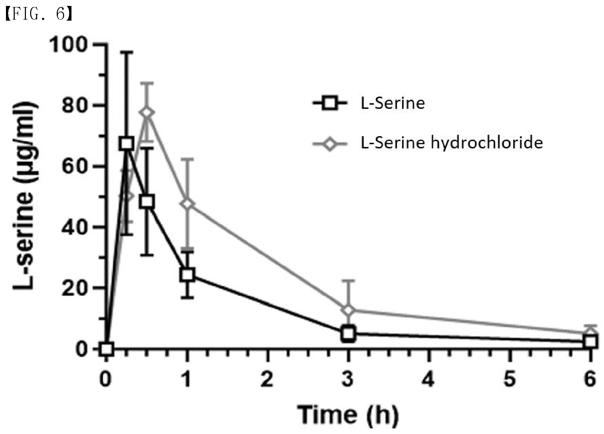

As a result, as shown in Table 1, it was confirmed that L-serine hydrochloride had a significantly increased half-life and blood drug concentration compared to L-serine (Fig. 6).

**[Table 1]**

| Parameter | L-Serine | L-Serine Hydrochloride (Example 3) |
|---|---|---|
| t_{1/2} (h) | 1.21±0.48 | 1.80±0.27 |
| Tₘₐₓ (h) | 0.25 | 0.62 |
| Cₘₐₓ (µg/mL) | 67.50±25.92 | 77.69±8.49 |
| AUC₀₋₆ (µg/mL*h) | 81.46±22.54 | 141.34±37.40 |
| Cl/F((mg/kg)/(µg/mL)/h) | 4.92±1.20 | 3.77±0.94 |

### Experimental Example 2

In order to perform a behavioral experiment on valproic acid (VPA)-induced autism model mice using L-serine hydrochloride prepared in Example 3, 600 mg/kg of VPA dissolved in 0.9% NaCl saline was administered subcutaneously to pregnant dams to obtain 14 VPA-induced autism model mice. Meanwhile, 8 pups born to the dams that were not administered VPA were used as a control group. For 14 days starting 21 days after birth, (i) the vehicle was orally administered to the control mice (n=8) (Ctrl + Vehicle treatment group), (ii) the vehicle was orally administered to some of the VPA-induced autism model mice (n=8) (VPA + Vehicle treatment group), and (iii) the L-serine hydrochloride prepared in Example 3 was orally administered to another some of the VPA-induced autism model mice (n=6) (VPA + L-serine hydrochloride treatment group). The drug was orally administered at a dose of 10 mL/kg per day based on the individual's body weight, which is equivalent, on a molar basis, to administering 400 mg/kg of L-serine hydrochloride to humans.

Thirty-five days after birth, the mice's social interaction abilities were tested using a three-chamber test. Social novelty was evaluated based on the interaction time during an initial encounter with a novel mouse compared with a re-encounter with a familiar mouse. Sociability reflects the degree of social interaction between a mouse and another mouse, and was measured by evaluating the interaction time that the mouse spent interacting with another mouse located in one side of an empty chmber.

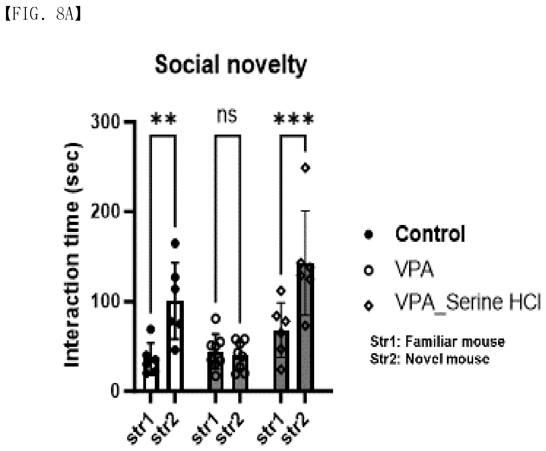

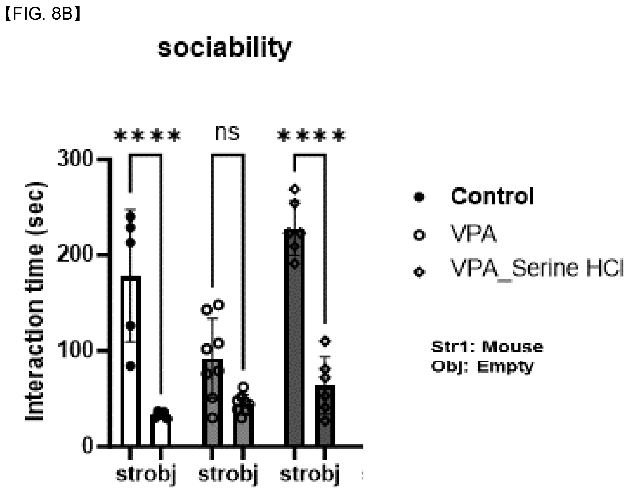

As a result, vehicle-treated VPA mice (VPA) showed significantly lower social novelty and sociability compared to vehicle-treated control mice (Control). In contrast, L-serine hydrochloride-treated VPA mice (VPA + L-serine hydrochloride) showed statistically significant improvements in both social novelty and sociability assessments, completely restoring social novelty and sociability to levels comparable to or exceeding those of the control level (Figs. 8A and 8B).

## Claims

1. A method for preparing L-serine hydrochloride, comprising:
(a) mixing L-serine of Formula 1 below with an organic solvent;
(b) adjusting the pH of the mixed solution of step (a) with hydrochloric acid diluted in an organic solvent;
(c) forming a salt by stirring the solution whose pH has been adjusted in step (b); and
(d) filtering the salt prepared in step (c) and then washing the excess hydrochloric acid with an organic solvent

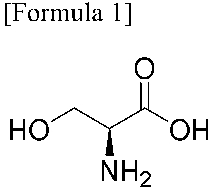

2. The preparation method of claim 1, wherein the organic solvent of step (a) is selected from the group consisting of acetone, methanol, ethanol, isopropanol, butanol, hexanol, ethyl acetate, toluene, methyl-*t*-butyl ether, and methylene chloride.

3. The preparation method of claim 1, wherein the pH in step (b) is adjusted to 1 to 5.

4. The preparation method of claim 1, wherein the organic solvent of step (b) is selected from the group consisting of acetone, methanol, ethanol, isopropanol, butanol, hexanol, ethyl acetate, toluene, methyl-*t*-butyl ether, and methylene chloride.

5. The preparation method of claim 1, wherein the stirring time in step (c) is 2 to 48 hours.

6. The preparation method of claim 1, wherein the stirring in step (c) is performed at a temperature at or below room temperature.

7. The preparation method of claim 1, wherein the organic solvent of step (d) is selected from the group consisting of acetone, methanol, ethanol, isopropanol, butanol, hexanol, ethyl acetate, toluene, methyl-*t*-butyl ether, methylene chloride, cyclohexane, and pentane.

8. The preparation method of claim 1, wherein the organic solvent of step (d) has a moisture content of 1% or less.

9. The preparation method of claim 1, wherein after step (d), the method further comprises step (e) of drying to remove the solvent.

10. The preparation method of claim 9, wherein in step (e), the drying temperature is between 20°C and 80°C.

11. L-serine hydrochloride of Formula 2 below prepared by the preparation method of claim 1:

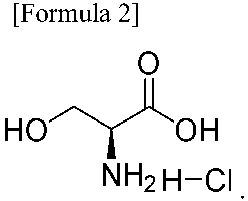

12. A pharmaceutical composition for the prevention or treatment of neurological, mental, and cognitive disorders, comprising L-serine hydrochloride of claim 11 as an active ingredient.

13. The pharmaceutical composition of claim 12, wherein the neurological, mental, and cognitive disorders are selected from the group consisting of autism spectrum, bipolar disorder, Tourette's syndrome, tics, Prader-Willi syndrome, attention deficit hyperactivity disorder (ADHD), fragile-X syndrome, dementia, Alzheimer's disease, Parkinson's disease, schizophrenia, depression, and memory impairment.

14. The pharmaceutical composition of claim 12, wherein the pharmaceutical composition is in any one formulation selected from the group consisting of tablets, lozenges, capsules, granules, powders, and liquids.

15. The pharmaceutical composition of claim 14, wherein in the tablet formulation, L-serine hydrochloride is comprised in an amount of 0.05 wt% to 95 wt% based on the total weight of the tablet.

16. A health functional food composition for the prevention or improvement of neurological, mental, and cognitive disorders, comprising L-serine hydrochloride of claim 11 as an active ingredient.

17. The health functional food composition of claim 16, wherein the neurological, mental, and cognitive disorders are selected from the group consisting of autism spectrum, bipolar disorder, Tourette's syndrome, tics, Prader-Willi syndrome, attention deficit hyperactivity disorder (ADHD), fragile-X syndrome, dementia, Alzheimer's disease, Parkinson's disease, schizophrenia, depression, and memory impairment.

18. A method for the prevention or treatment of neurological, mental, and cognitive disorders, comprising administering L-serine hydrochloride of claim 11 to a subject.

19. Use of L-serine hydrochloride of claim 11 for the prevention or treatment of neurological, mental, and cognitive disorders.

20. Use of L-serine hydrochloride of claim 11 in the manufacture of a medicament for the prevention or treatment of neurological, mental, and cognitive disorders.
